# EUROPEAN PATENT APPLICATION

(11) **EP 3 098 742 A1**
(43) Date of publication of application: **30.11.2016**
(21) Application number: 15305791.4
(22) Date of filing: 26.05.2015
(51) Int. Cl.: G06F 19/22, G06N 3/12

(54) **METHOD AND APPARATUS FOR CREATING A PLURALITY OF OLIGOS WITH A TARGETED DISTRIBUTION OF NUCLEOTIDE TYPES**

(71) Applicant: Thomson Licensing, 92130 Issy-les-Moulineaux (FR)
(72) Inventor: Huetter, Ingo, 30982 Pattensen (DE); Blawat, Meinolf, 30659 Hannover (DE); Gaedke, Klaus, 30659 Hannover (DE); Chen, Xiaoming, 30165 Hannover (DE)
(74) Representative: Huchet, Anne

(57) **Abstract**

Methods and apparatuses for creating oligos with a targeted nucleotide type distribution, and for restoring the initially encoded data, are presented. A method of creating oligos with a targeted of nucleotide type distribution, represented by sequences of quaternary code symbols, comprises -acquiring (14) an unmodified sequence, -selecting (15) a modification rule from a set of different modification rules, - generating (16) a modified sequence from the unmodified sequence, and -creating (17) an oligo comprising a nucleotide sequence corresponding to the modified symbol sequence. The corresponding method of determining unmodified code symbol sequences from oligos generated from modified code symbol sequences comprises - determining (64) a modified code symbol sequence by sequencing a corresponding oligo, -determining (65) a selected modification rule of a rule set that has been applied to generate the modified sequence, and -generating (66) an unmodified sequence from the modified sequence using a reverse modification rule.

## Description

### FIELD

The present disclosure relates to storing/archiving user data in nucleic acid molecules, i.e. oligos, and to the corresponding data retrieval. Methods and apparatuses for creating a plurality of oligos with a targeted distribution of nucleotide types, as well as for restoring the initially encoded data, are presented. In particular, the present principles relate to a method and apparatus for creating a plurality of oligos with a targeted distribution of nucleotide types, represented by a plurality of sequences of quaternary code symbols, wherein each code symbol value is represented by a nucleotide type, and to a corresponding method and apparatus for determining unmodified sequences of quaternary code symbols from a plurality of oligos generated from modified code symbol sequences.

### BACKGROUND

A nucleic acid is a polymeric macromolecule and consists of a sequence of monomers known as nucleotides. A nucleotide comprises a sugar component, a phosphate group and a nitrogenous base or nucleobase. Nucleic acid molecules where the sugar component of the nucleotides is deoxyribose are DNA (deoxyribonucleic acid) molecules, whereas nucleic acid molecules where the sugar component of the nucleotides is ribose are referred to as RNA (ribonucleic acid) molecules. DNA and RNA are biochemical storage molecules of genetic information appearing in living organisms.

The nucleic acid molecules are assembled as chains or strands of nucleotides. For instance, RNA molecules are single helical nucleic strands, while DNA double helix molecules are formed out of two antiparallel nucleic strands. In contrast to RNA molecules, DNA molecules are usually more robust to biological, biochemical and biophysical stress, like enzymes, acids and heat. Nucleic acid molecules can be generated artificially and their chain structure can be used for encoding any kind of user data by a biological, biochemical and/or biophysical process using a nucleic acid synthesizer. For storing data in synthesized, i.e. artificially created, DNA or RNA, usually comparably short DNA or RNA molecules (oligonucleotides, short: oligos) are generated. With these oligos, a data storage system can be realized. The oligos, i.e. the synthesized nucleic acid molecules, carry the information encoded by the succession of the different nucleotides forming the oligos. The succession or sequence of the nucleotides encodes a sequence of code words, each consisting of a set of code symbols and encoding any kind of user data, e.g. video, audio, metadata, program code etc.

Biochemical storage of arbitrary digital information in DNA (Deoxyribonucleic Acid) oligos has been investigated in "Next-generation digital information storage", Church et al., Science, Vol. 337, 2012 [I] and in "Towards practical, high-capacity, low-maintenance information storage in synthesized DNA", Goldman et al., Nature, Vol. 494, 2013 [II].

DNA strands are built from four different nucleotides identified by their respective nucleobases or nitrogenous bases, namely, Adenine, Thymine, Cytosine and Guanine, which are denoted shortly as A, T, C and G, respectively. As another example, RNA strands also consist of four different nucleotides identified by their respective nucleobases, namely, Adenine, Uracil, Cytosine and Guanine, which are denoted shortly as A, U, C and G, respectively.

With the four different types of nucleotides, in principle one nucleotide can be used to store two bits, i.e. two binary code symbols, which may be represented as one quaternary code symbol. User data to be stored in oligos can, therefore, be provided as one or more sequences of quaternary code symbols, such that each of the four different values of the quaternary code symbol can be directly mapped to a corresponding one of the four different nucleotide types. For example, code symbol values can be assigned to the four types of DNA nucleotides, identified by their nucleobases, as follows: 0 to G, 1 to A, 2 to T, and 3 to C. Any other unambiguous assignment may be used. Code symbol values may, for example, also be represented using letters, such as g, a, t, c.

Today's synthesizers for creating artificial sequences of nucleotides, i.e. oligos, can only produce oligos of a limited size, and, therefore, the user data information to be stored is usually divided into several small pieces. Each piece is then stored within one oligo, which contains some additional information for identifying the oligo.

The stored information can be retrieved from the stored oligos using a sequencer, which carries out a process of determining the order and type of nucleotides within the oligo. Then the read out order of nucleotides is processed or decoded to recover the original user data stored in the oligo.

If "real world" user data are stored in oligos, it will sometimes happen that there is a high correlation within the data (e.g. several times the same number to be stored), leading to long sequences with an unequal distribution of code symbols, respectively to a low diversity of the four different nucleotide types in the corresponding synthesized oligos, which is in fact an unequal distribution of the nucleotide types. Sequencers may not always function perfectly in such cases, as can be seen, for example, in "Low-Diversity Sequencing on the Illumnia HiSeq Platform", Illumina, Technical Note: DNA Sequencing, 2014 [III] and "Using a PhiX Control for HiSeq® Sequencing Runs", Illumina, Technical Note: Sequencing, 2013 [IV].

Therefore, a technique is needed to prevent such unequal distributions. This may be accomplished by using coding schemes with very high oligo coverages or inefficient coding techniques, but due to introduction of high redundancy, such solutions may be expensive due to increasing synthesizing costs.

There remains a need for an oligo creation scheme that allows an oligo nucleotide distribution modification to at least partly avoid unequal distribution of nucleotide types across multiple synthesized oligos without introducing additional redundancy.

### SUMMARY

A method and apparatus for creating a plurality of oligos with a targeted distribution of nucleotide types without introducing extra redundancy, as well as a corresponding method and apparatus for determining unmodified sequences of quaternary code symbols from a plurality of oligos where the nucleotide type distribution has been modified, according to the appended claims are suggested.

According to one aspect of the present principles, a method of creating a plurality of oligos with a targeted distribution of nucleotide types, represented by a plurality of sequences of quaternary code symbols, each code symbol value representing a, i.e. a different, nucleotide type, comprises the following:
for the plurality of sequences of quaternary code symbols:
   - acquiring an unmodified sequence from the plurality of sequences;
   - selecting a modification rule from a set of different modification rules, each adapted to unambiguously assign unmodified code symbol values to corresponding modified code symbol values;
   - generating a modified sequence from the unmodified sequence, i.e. transforming the unmodified sequence into a modified sequence, wherein the unmodified code symbol values of the unmodified sequence are replaced with their assigned corresponding modified code symbol values according to the selected modification rule, if said unmodified values differ from said assigned corresponding modified values; and
   - creating an oligo of the plurality of oligos, comprising a nucleotide sequence corresponding to the modified sequence of quaternary code symbols.

The method may, for example, be computer implemented, wherein the oligo creation is performed using, e.g., a nucleic acid synthesizer.

The selecting is performed depending on the targeted distribution. The targeted distribution will often be an equal distribution, i.e. it may be desired to create a plurality of oligos wherein each of the nucleotide types occurs approximately equally often within the plurality of oligos. However, in other embodiments, other distributions can be desired, e.g. depending on the availability of nucleotides for the synthesizing process.

The term "plurality of sequences of quaternary code symbols, each code symbol value representing a nucleotide type" refers to user data already available in a quaternary representation or transcoded from a binary representation, i.e. sequences of bits, to a quaternary representation suitable to be encoded in oligos, i.e. suitable to create nucleotide sequences from, wherein each value of the quaternary code symbol (e.g. 0, 1, 2, 3) can be assigned to a corresponding different nucleotide type (e.g. A, T, C, G for DNA).

An "unmodified sequence" is a sequence of the plurality of sequences of quaternary code symbols as originally provided, i.e. before modification by application of the described method. If, for example, the user data is initially provided as a set of binary sequences, quaternary sequences generated from the binary sequences are still "unmodified sequences", unless the method of creating a plurality of oligos with a targeted distribution of nucleotide types has been applied, and may again be considered the "unmodified sequences" after the applied modification has been reverted.

A modification rule specifies a direct mapping of unmodified code symbol values to modified code symbol values. Unmodified and modified quaternary code symbols share the same domain, e.g. {0, 1, 2, 3} or {a, t, c, g} or {A, T, C, G}. Application of the rule results in replacement of each code symbol of unmodified value by a code symbol of the modified value. In one embodiment, the modification rules are provided, e.g., by means of a look-up table stored in a memory.

Accordingly, an apparatus for creating a plurality of oligos with a targeted distribution of nucleotide types, represented by a plurality of sequences of quaternary code symbols, each code symbol value representing a nucleotide type, comprises
- an acquisition unit configured to acquire unmodified sequences from the plurality of sequences;
- a selection unit configured to select, for each of the plurality of sequences of quaternary code symbols, a modification rule from a set of different modification rules, each adapted to unambiguously assign unmodified code symbol values to corresponding modified code symbol values;
- a modification unit configured to generate a modified sequence from the unmodified sequence, wherein the unmodified code symbol values of the unmodified sequence are replaced with their assigned corresponding modified code symbol values according to the selected modification rule, if said unmodified values differ from said assigned corresponding modified values; and
- an oligo creation unit configured to create an oligo of the plurality of oligos, comprising a nucleotide sequence corresponding to the modified sequence of quaternary code symbols.

The selection unit is configured to select said modification rule depending on the targeted distribution.

In one embodiment, the acquisition unit comprises an interface to a memory containing the plurality of sequences generated in advance. In another embodiment, the acquisition unit comprises, e.g., a code symbol sequence generator or encoder for encoding the user data as unmodified sequences of quaternary code symbols.

Different embodiments of the apparatus for creating a plurality of oligos with targeted distribution of nucleotide types are available: The acquisition unit, the selection unit, the modification unit and the oligo creation unit can be provided as separate devices or jointly as one device. The acquisition unit, the selection unit and the modification unit may also be provided as functionality carried out or implemented by a processor, microprocessor, microcontroller or other processing device, computer or other programmable apparatus connected to or comprising a memory and connected with the oligo creation unit. The oligo creation unit may be, or be comprised in, a nucleic acid synthesizer, for example a DNA synthesizer.

In another embodiment, an apparatus for creating a plurality of oligos with a targeted distribution of nucleotide types, represented by a plurality of sequences of quaternary code symbols, each code symbol value representing a nucleotide type, comprises a processing device, a nucleic acid synthesizer device and a memory device having stored therein instructions, which, when executed by the processing device, cause the apparatus for the plurality of sequences of quaternary code symbols to:
- acquire an unmodified sequence from the plurality of sequences;
- select a modification rule from a set of different modification rules, each adapted to unambiguously assign unmodified code symbol values to corresponding modified code symbol values;
- generate a modified sequence from the unmodified sequence, wherein the unmodified code symbol values of the unmodified sequence are replaced with their assigned corresponding modified code symbol values according to the selected modification rule, if said unmodified values differ from said assigned corresponding modified values; and
- create an oligo of the plurality of oligos, comprising a nucleotide sequence corresponding to the modified sequence of quaternary code symbols.

The instructions cause the apparatus to select said modification rule depending on the targeted distribution.

Further, in one embodiment a computer program comprises instructions enabling control of a creation of a plurality of oligos with a targeted distribution of nucleotide types, represented by a plurality of sequences of quaternary code symbols, each code symbol value representing a nucleotide type, which, when executed by a computer, cause the computer for the plurality of sequences of quaternary code symbols to:
- acquire an unmodified sequence from the plurality of sequences;
- select a modification rule from a set of different modification rules, each adapted to unambiguously assign unmodified code symbol values to corresponding modified code symbol values;
- generate a modified sequence from the unmodified sequence, wherein the unmodified code symbol values of the unmodified sequence are replaced with their assigned corresponding modified code symbol values according to the selected modification rule, if said unmodified values differ from said assigned corresponding modified values; and
- actuate an oligo creation unit, such as a nucleic acid synthesizer device, connectable to the computer to create an oligo of the plurality of oligos, comprising a nucleotide sequence corresponding to the modified sequence of quaternary code symbols.

Further, according to one aspect of the present principles, a method of storing user data in a plurality of nucleic acid molecules (oligos) with a targeted distribution of nucleotides types, said oligos comprising sequences of nucleotides represented by sequences of quaternary code symbols, each code symbol value representing a nucleotide type, comprises:
- accessing user data;
- generating a plurality of unmodified sequences of quaternary code symbols from said user data;
- transforming said plurality of unmodified sequences into a plurality of modified sequences; and
- creating a plurality of oligos from said plurality of modified sequences by mapping each quaternary code symbol to a nucleotide, wherein said plurality of oligos stores said user data.

According to a further aspect of the present principles, a method of determining unmodified sequences of quaternary code symbols from a plurality of oligos generated from modified code symbol sequences with code symbol values representing nucleotide types comprises the following:
for at least some of the plurality of oligos:
   - determining a modified sequence of quaternary code symbols by sequencing a corresponding oligo of the plurality of oligos;
   - determining a selected modification rule of a set of modification rules that has been applied to generate the modified sequence, each modification rule adapted to unambiguously assign unmodified code symbol values to corresponding modified code symbol values; and
   - generating an unmodified sequence of quaternary code symbols from the modified sequence, i.e. transforming the modified sequence into an unmodified sequence, wherein the modified code symbol values of the modified sequence are replaced with their assigned corresponding unmodified code symbol values according to a reverse modification rule adapted to revert changes that have been applied by the selected modification rule.

The method for determining unmodified sequences may, for example, be computer implemented, wherein the sequencing is performed using, e.g., a nucleic acid sequencer.

Accordingly, an apparatus for determining unmodified sequences of quaternary code symbols from a plurality of oligos generated from modified code symbol sequences with code symbol values representing nucleotide types comprises
- a sequencer unit configured to determine a modified code symbol sequence by sequencing a corresponding oligo of the plurality of oligos;
- a modification rule determination unit configured to determine a selected modification rule of a set of modification rules that has been applied to generate the modified code symbol sequence, each modification rule adapted to unambiguously assign unmodified code symbol values to corresponding modified code symbol values; and
- a modification removal unit configured to generate, for each of at least some of the plurality of oligos, an unmodified sequence from the modified sequence, wherein the modified code symbol values of the modified sequence are replaced with their assigned corresponding unmodified code symbol values according to a reverse modification rule adapted to revert changes that have been applied by the selected modification rule.

Different embodiments of the apparatus for determining unmodified sequences are available: The modification rule determination unit and the modification removal unit can be provided as separate devices or jointly as one device. The modification rule determination unit and the modification removal unit may also be provided as functionality carried out or implemented by a processor, microprocessor, microcontroller or other processing device, computer or other programmable apparatus connected to or comprising a memory and connected with the sequencer unit. The sequencer unit may be, or be comprised in, a nucleic acid sequencer, for example a DNA sequencer.

In another embodiment, an apparatus for determining unmodified sequences of quaternary code symbols from a plurality of oligos generated from modified code symbol sequences with code symbol values representing nucleotide types comprises a processing device, a nucleic acid sequencer device and a memory device having stored therein instructions, which, when executed by the processing device, cause the apparatus for at least some of the plurality of oligos to:
- determine a modified sequence of quaternary code symbols by sequencing a corresponding oligo of the plurality of oligos;
- determine a selected modification rule of a set of modification rules that has been applied to generate the modified sequence, each modification rule adapted to unambiguously assign unmodified code symbol values to corresponding modified code symbol values; and
- generate an unmodified sequence of quaternary code symbols from the modified sequence, wherein the modified code symbol values of the modified sequence are replaced with their assigned corresponding unmodified code symbol values according to a reverse modification rule adapted to revert changes that have been applied by the selected modification rule.

Further, in one embodiment, a computer program comprises instructions enabling control of a determining of unmodified sequences of quaternary code symbols from a plurality of oligos generated from modified code symbol sequences with code symbol values representing nucleotide types, which, when executed by a computer, cause the computer for at least some of the plurality of oligos to:
- determine a modified sequence of quaternary code symbols by actuating a sequencer unit connectable to the computer to sequence a corresponding oligo of the plurality of oligos;
- determine a selected modification rule of a set of modification rules that has been applied to generate the modified sequence, each modification rule adapted to unambiguously assign unmodified code symbol values to corresponding modified code symbol values; and
- generate an unmodified sequence of quaternary code symbols from the modified sequence, wherein the modified code symbol values of the modified sequence are replaced with their assigned corresponding unmodified code symbol values according to a reverse modification rule adapted to revert changes that have been applied by the selected modification rule.

According to a further aspect of the present principles, a method of retrieving (or extracting or detecting) user data stored in a plurality of nucleic acid molecules (oligos) with a targeted distribution of nucleotides types, said oligos comprising sequences of nucleotides represented by sequences of quaternary code symbols, each code symbol value representing a nucleotide type, comprises:
- accessing the plurality of oligos;
- retrieving modified sequences from said oligos;
- inverse (or reverse) transforming said modified sequences into unmodified sequences; and
- retrieving said user data from said unmodified sequences.

The provided solution allows modification of a frequency distribution of nucleotide types across multiple oligos depending on an application of a set of modification rules in view of a targeted modified distribution to be achieved or at least approximated.

The provided approach at least has the effect that the frequency distribution of nucleotide types can be modified, e.g. to achieve improved suitability of synthesized oligos for reliable sequencing without increased synthesizing costs caused by introduction of additional redundancy to the code symbol sequences before synthesizing oligos from them.

For creating a plurality of oligos with a targeted distribution of nucleotide types, in one embodiment the set of modification rules comprises 4 modification rules that differ from each other in that each unmodified code symbol value is assigned to a different corresponding modified code symbol value. In other words, the modification rules contained in the set of modification rules are not correlated with each other. If, for example, the targeted distribution of nucleotide types is an equal distribution, i.e. nucleotides of each of the four nucleotide types should occur in a plurality of synthesized oligos approximately equally often, this can be achieved by applying the different modification rules equally often when modifying each of the unmodified quaternary code symbol sequences using one of the modification rules. This may, for example, help avoid long sections or runs of consecutive nucleotides of a same type.

In one embodiment the selecting comprises randomly selecting the modification rule from the set of different modification rules. This may result in application of each of the modification rules equally often, given that the amount of code symbol sequences to be modified before generation of corresponding synthesized oligos is high. The modulation rule randomly chosen for modification can later be determined, for example, by checking the reconstructed code symbol sequences for correctness for all possible corresponding reverse modulation rules.

In another embodiment the selecting comprises pseudo randomly selecting the modification rule from the set of different modification rules. For example, the different modulation rules may be selected periodically according to a modulo 4 division of the number of the unmodified code symbol sequence currently being modified. For example, for a set of uncorrelated modification rules this also allows generation of a plurality of oligos exhibiting an approximately equal distribution of nucleotide types. The selected modulation rule may be determined during demodulation after sequencing as with a purely random modulation rule selection scheme. In one embodiment where a consecutive numbering of the created oligo is encoded within the oligo, the applied modulation rule can also be determined from a modulo 4 division of said encoded oligo number.

In another embodiment the selecting comprises selecting the modification rule from the set of different modification rules based on an evaluation of a parameter of the unmodified code symbol sequence invariant to modification by any of the modification rules. The idea behind this deterministic approach is to use attributes of the code symbol sequences, which are used to choose the modification rule that shall be applied. If these attributes remain unchanged after applying the rule, it is possible to also determine which inverse rule shall be applied to reverse the modification. This has the advantage that it is not necessary to first try all possible rules and to then finally select the correct one, but it is possible to directly choose the correct one.

In one example embodiment, said parameter is a number of pairs of neighboring code symbols having identical code symbol values. In another example embodiment, a number of triples of neighboring code symbols having identical code symbol values is chosen as said parameter invariant to modification.

In an example embodiment, each of said modification rules is assigned to a different interval of values of said parameter, said different interval depending on a frequency distribution of the values of said parameter. For example, in order to achieve an equal distribution of 4 nucleotide types, the frequency distribution of the values of said parameter is divided into 4 intervals such that the sums of occurrences of the parameter values covered by the corresponding intervals, i.e. the integrals of the frequency distribution over the intervals, are identical.

For determining unmodified sequences of quaternary code symbols from a plurality of oligos generated from modified code symbol sequences with code symbol values corresponding to nucleotide types, in one embodiment the determining of the selected modification rule and generating an unmodified sequence comprises
- generating a set of potential unmodified sequences based on reverse modification rules corresponding to all modification rules of the set of modification rules;
- evaluating compliance of the potential unmodified sequences with one or more constraints a correct unmodified sequence complies with before modification; and
- determining the selected modification rule as the modification rule that allows to generate the correct unmodified sequence in compliance with the one or more constraints. In other words, for determining the correct rule that has been applied to modify the original modified sequence, reverse or inverse modification rules, each corresponding to one of the modification rules, are provided, and all inverse rules are applied to a modified sequence of quaternary code symbols determined by sequencing an oligo to generate a corresponding set of potential unmodified sequences. Then it is checked, which of the resulting potential code symbol sequences contains only valid code words - as the probability that a wrong rule will lead to a code symbol sequence with all code words being valid is extremely low. In one embodiment, if the received oligos are not error free due to the influence of the "channel", i.e. due to errors introduced during synthesizing, storage, and/or sequencing of the oligos carrying the encoded user data, the reverse modification rule allowing creation of a potential unmodified sequence with the maximum amount of valid code words is chosen as the reverse of the rule that has been used as the selected modification rule.

In one example embodiment said one or more constraints comprises at least one of a maximum length of a self-reverse complementary part of an oligo corresponding to the unmodified code symbol sequence, a maximum run length of identical code symbols within the unmodified code symbol sequence and a combination thereof. These upper limits are applied to the synthesis process in order to avoid synthesis of long self-reverse complementary nucleic acid sequences and run lengths of identical nucleotides beyond a certain limit, as otherwise correctness of the sequencing of the synthesized oligos is reduced.

In another embodiment said determining of the selected modification rule comprises determining the selected modification rule from the set of different modification rules based on an evaluation of a parameter of the modified code symbol sequence invariant to modification by any of the modification rules. If the modification rule has been selected based on an evaluation of a parameter of the modified code symbol sequence invariant to modification by any of the modification rules, it is possible to determine, which modification rule has been applied and which corresponding inverse (i.e. reverse) rule shall now be applied to reverse the modification.

In one example embodiment said parameter is a number of pairs of neighboring code symbols having identical code symbol values. In another example embodiment a number of triples of neighboring code symbols having identical code symbol values is said parameter invariant to modification.

In one example embodiment each of the modification rules is assigned to a different interval of values of said parameter, wherein the different interval depends on a frequency distribution of said values of said parameter. The method comprises
- evaluating at least some of the modified code symbol sequences to determine the frequency distribution of said values of said parameter;
- determining the different intervals assigned to the modification rules; and
- determining the selected modification rule by evaluating the modified code symbol sequence with respect to the different intervals.

While not explicitly described, the presented embodiments may be employed in any combination or sub-combination.

### BRIEF DESCRIPTION OF THE DRAWINGS

- Fig. 1: schematically illustrates an embodiment of a method for creating a plurality of oligos with a targeted distribution of nucleotide types from a plurality of sequences of quaternary code symbols with code symbol values corresponding to nucleotide types;
- Fig. 2: schematically illustrates an example of a possible frequency distribution of unmodified code symbol sequences over contained number of pairs of neighboring code symbols of identical value;
- Fig. 3: schematically illustrates an example of a possible assignment of modification rules to intervals introduced to a frequency distribution of unmodified code symbol sequences over contained number of pairs of neighboring code symbols of identical value;
- Fig. 4: schematically illustrates an embodiment of an apparatus for creating a plurality of oligos with a targeted distribution of nucleotide types from a plurality of sequences of quaternary code symbols with code symbol values corresponding to nucleotide types;
- Fig. 5: schematically illustrates another embodiment of an apparatus for creating a plurality of oligos with a targeted distribution of nucleotide types from a plurality of sequences of quaternary code symbols with code symbol values corresponding to nucleotide types;
- Fig. 6: schematically illustrates an embodiment of a method for determining unmodified sequences of quaternary code symbols from a plurality of oligos generated from modified code symbol sequences with code symbol values corresponding to nucleotide types;
- Fig. 7: schematically illustrates an example embodiment of determining of the selected modification rule and generating an unmodified sequence according to the method for determining unmodified sequences of quaternary code symbols from a plurality of oligos generated from modified code symbol sequences with code symbol values corresponding to nucleotide types;
- Fig. 8: schematically illustrates an embodiment of an apparatus for determining unmodified sequences of quaternary code symbols from a plurality of oligos generated from modified code symbol sequences with code symbol values corresponding to nucleotide types;
- Fig. 9: schematically illustrates another embodiment of an apparatus for determining unmodified sequences of quaternary code symbols from a plurality of oligos generated from modified code symbol sequences with code symbol values corresponding to nucleotide types; and
- Fig. 10: schematically illustrates an embodiment of a nucleic acid storage system.

### DETAILED DESCRIPTION OF EMBODIMENTS

For a better understanding of the principles, example embodiments are explained in more detail in the following description with reference to the figures. It is understood that the present solution is not limited to these exemplary embodiments and that specified features can also expediently be combined and/or modified without departing from the scope of the present principles as defined in the appended claims.

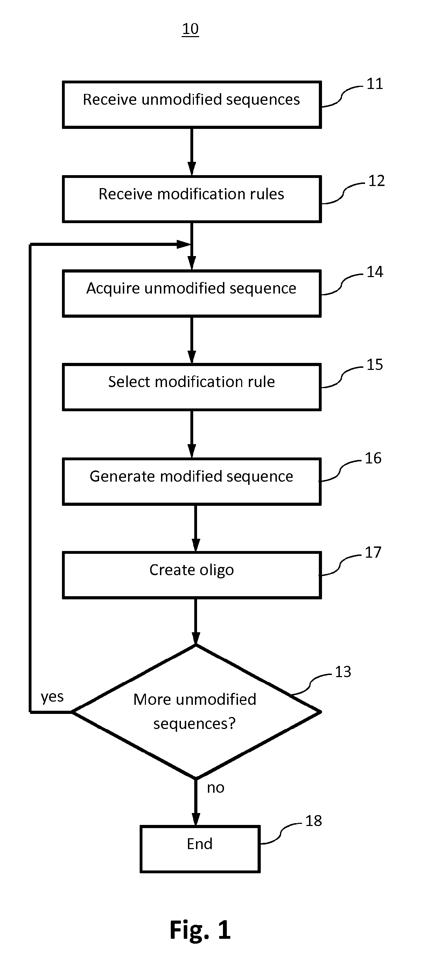

Referring to Fig. 1, one embodiment of a method 10 of creating a plurality of oligos with a targeted distribution of nucleotide types, represented by a plurality of sequences of quaternary code symbols, each code symbol value representing a, i.e. a different, nucleotide type, is schematically shown. The method may, for example, be computer implemented, wherein the oligo creation is performed using, e.g., a nucleic acid synthesizer.

In the shown embodiment, in a first step 11 a plurality of unmodified sequences of quaternary code symbols, grouped into sequences of code words, is received. These unmodified code symbol sequences carry encoded user data to be stored in a plurality of oligos.

Further, in a second step 12 a set of modification rules is received, each adapted to unambiguously assign unmodified code symbol values, i.e. values of unmodified code symbols, to corresponding modified code symbol values, i.e. corresponding values of modified code symbols. Furthermore, in one embodiment the targeted distribution of nucleotide types is preselected, e.g. as an equal distribution. In another embodiment the targeted distribution is received as a parameter value.

The following steps are repeated for each of the plurality of sequences of quaternary code symbols. This is achieved, for example, by checking 13 after processing steps 14, 15, 16 and 17 whether or not any of the plurality of quaternary code symbol sequences has not yet been modified and repeating the steps if more sequences of unmodified code symbols remain.

In step 14 an unmodified sequence, i.e. a sequence of unmodified quaternary code symbols, is acquired from the plurality of sequences.

In a next step 15 a modification rule is selected from the set of different modification rules. The selection of the modification rule is performed depending on the targeted distribution. If, for example, an equal distribution of nucleotide types within the oligos to be created is desired, the modification of the code symbol sequences (that will be used to create oligos with a corresponding nucleotide sequences from) is done in such a way, that the mean rate of the four nucleotide types in the created oligos is about 0.25 - even if the rate of the incoming unmodified quaternary code symbols shows a low diversity.

For the shown embodiment four modification rules are used that replace (if necessary) all code symbols of one (unmodified) value with the code symbols of another (modified) value. Each of the modification rules does the replacement in a different way. Table 1 below gives the details of an example replacement, wherein, for enhanced clarity how the exchange of code symbol values affects the corresponding nucleotide types for generation of corresponding oligos, the code symbol values are represented in line with DNA nucleotide types, i.e. as A, C, G and T:

**Table 1**

| Original symbol value | Replaced by | | | |
|---|---|---|---|---|
| | rule #1 | rule #2 | rule #3 | rule #4 |
| A | A | T | G | C |
| C | C | A | T | G |
| G | G | C | A | T |
| T | T | G | C | A |

In a next step 16 a modified sequence is generated from the unmodified sequence, wherein the unmodified code symbol values of the unmodified sequence are replaced with their assigned corresponding modified code symbol values according to the selected modification rule, if said unmodified values differ from said assigned corresponding modified values.

In a next step 17 an oligo of the plurality of oligos is created, comprising a nucleotide sequence corresponding to the modified sequence of quaternary code symbols.

Depending on the result of the checking 13, steps 14, 15, 16 and 17 are either repeated with a next unmodified sequence or the method proceeds to an end step 18. The end step 18 may, for example, refer to further processing or storing the created oligo in a nucleic acid storage for later retrieval and decoding.

With every repetition, a modification rule of the set of modification rules is selected in step 15 for each unmodified code symbol sequence according to a random selection scheme, e.g. using a random generator. Applying these rules leads to a more equal distribution of nucleotides. If e.g. in several unmodified code symbol sequences the "A" code symbol value occurs much more often than the other code symbol values, after applying the modification rules, the "A" (#1), "T" (#2), "G" (#3) or "C" (#4) code symbol will occur most often - which will statistically lead to an equal probability for each code symbol and, therefore, nucleotide type in the created oligos, for example:
AAACAAAGTAAAAC → (rule #1) → AAACAAAGTAAAAC
GATAAATAAACAAT →(rule #2) → CTGTTTGTTTATTG
ATAATGAAACAAAT →(rule #3) → GCGGCAGGGTGGGC
TAAGAAATATAAAC → (rule #4) → ACCTCCCACACCCG

In one embodiment, instead of randomly selecting the modification rule, a pseudo random selection scheme is applied. Here, a simple algorithm, such as a modulo 4 division is used to choose the modification rule.

In another embodiment, the modification rule is selected according to a deterministic selection scheme. Here, one of the modification rules is chosen based on the content of the unmodified code symbol sequence. Attributes of the unmodified code symbol sequence, which remain unchanged after applying the rule are used to choose the rule that shall be applied. As those attributes remain unchanged, it is possible to also determine, which reverse modification rule shall be applied to do the reverse modification. This has the advantage that it is not necessary to first try all possible rules and then finally select the correct one, but it is possible to directly choose the correct one. For instance, possible attributes that are not changed by the rules are e.g. the number of pairs of consecutive code symbols of identical values within one code symbol sequence or the number of triples of consecutive code symbols of identical value in one code symbol sequence.

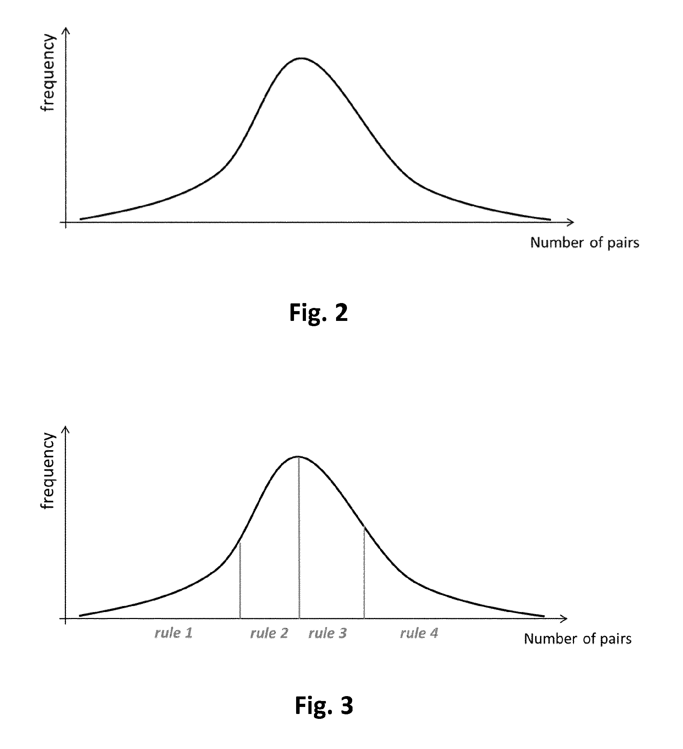

Without limiting the generality, in one embodiment where the number of pairs of neighboring code symbols of identical value is used to choose the rule, first the number of code symbol pairs of identical value within one code symbol sequence in a large number of code symbol sequences are counted to measure a frequency distribution, which may presumably look similar to the graph schematically shown in Fig. 2., which schematically illustrates an example of a possible frequency distribution of unmodified code symbol sequences over contained number of pairs of neighboring code symbols of identical value.

Referring to Fig. 3, a corresponding example of a possible assignment of modification rules to intervals introduced to a frequency distribution of unmodified code symbol sequences over contained number of pairs of neighboring code symbols of identical value is schematically illustrated. As shown in Fig. 3, the "Number of pairs" axis of the graph shown in Fig. 2 is divided into four intervals such that the areas below the curve are of similar size. These areas symbolize the integration of the measured frequency, i.e. relative occurrence, over different numbers of pairs, so the size of the areas represents a measure how often "numbers of pairs" of a given interval exist. Then, one of the four modification rules is assigned to each interval, leading to the effect that each rule is chosen with nearly the same probability. As the frequency distribution of the number of pairs is invariant to the modification, on the reverse modification side the same rule can be chosen like on the modification side.

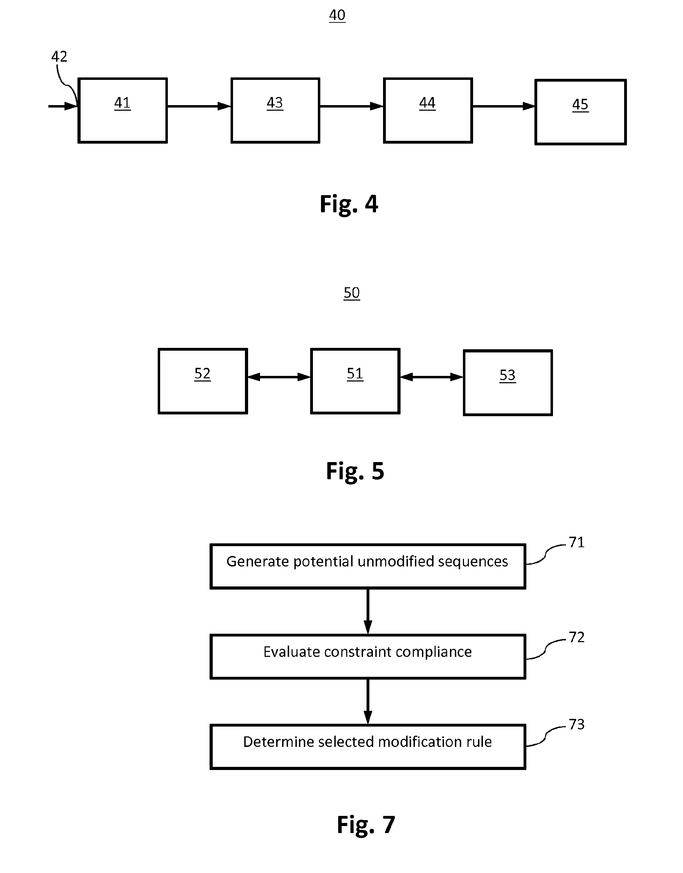

Accordingly, one embodiment of an apparatus 40 for creating a plurality of oligos with a targeted distribution of nucleotide types, represented by a plurality of sequences of quaternary code symbols, each code symbol value representing a nucleotide type, is schematically shown in Fig. 4. The apparatus 40 allows implementing the advantages and characteristics of the described method as part of an apparatus for creating a plurality of oligos with a targeted distribution of nucleotide types from a plurality of sequences of quaternary code symbols.

The apparatus 40 comprises an acquisition unit 41 configured to acquire unmodified sequences from the plurality of sequences. The acquisition unit 41 comprises an input 42 configured to receive the plurality of quaternary code symbol sequences. In one embodiment the input 42 is configured to receive binary encoded user data and the acquisition unit 41 is configured to also generate the plurality of quaternary code symbol sequences.

The apparatus 40 further comprises a selection unit 43 configured to select, for each of the plurality of sequences of quaternary code symbols, a modification rule from a set of different modification rules. Each of the different modification rules is adapted to unambiguously assign unmodified code symbol values to corresponding modified code symbol values. The modification rule is selected depending on the targeted distribution.

The apparatus 40 further comprises a modification unit 44 connected to receive the unmodified code symbol sequence acquired by the acquisition unit 41 and the modification rule selected by the selection unit 43. The modification unit 44 is configured to generate a modified sequence from the unmodified sequence, wherein the unmodified code symbol values of the unmodified sequence are replaced with their assigned corresponding modified code symbol values according to the selected modification rule, if said unmodified values differ from said assigned corresponding modified values.

Furthermore, the apparatus 40 comprises an oligo creation unit 45 connected to receive the modified sequence of quaternary code symbols. The oligo creation unit 45 is configured to create an oligo of the plurality of oligos, comprising a nucleotide sequence corresponding to the modified sequence of quaternary code symbols. The oligo creation unit 45 is, or is comprised in, a nucleic acid synthesizer, for example a DNA synthesizer.

In the embodiment shown in Fig. 4 the acquisition unit 41, the selection unit 43, the modification unit 44 and the oligo creation unit 45 directly communicate with each other. In another embodiment the apparatus comprises a controller unit connected to one or more of the units and controls their communication.

Depending on the embodiment, the acquisition unit 41, the selection unit 43, the modification unit 44 and the oligo creation unit 45 are provided as separate devices or jointly as one device. In one embodiment, the acquisition unit 41, the selection unit 43 and the modification unit 44 are provided as functionality carried out or implemented by a processor, microprocessor, microcontroller or other processing device, computer or other programmable apparatus connected to or comprising a memory and connected with the oligo creation unit 45.

Another embodiment of an apparatus 50 for creating a plurality of oligos with a targeted distribution of nucleotide types from a plurality of sequences of quaternary code symbols with code symbol values corresponding to nucleotide types according to the present principles is schematically illustrated in Fig. 5. The apparatus 50 comprises a processing device 51, a nucleic acid synthesizer device 52 and a memory device 53 storing instructions that, when executed, cause the apparatus to perform steps according to one of the described methods.

For example, the processing device 51 can be a processor adapted to perform the steps according to one of the described methods. In one embodiment according to the present principles, said adaptation comprises that the processor is configured, e.g. programmed, to perform steps according to one of the described methods of creating a plurality of oligos with a targeted distribution of nucleotide types, represented by a plurality of sequences of quaternary code symbols, each code symbol value representing a nucleotide type.

A part of the memory device 53 is a non-transitory program storage device readable by the processing device 51, tangibly embodying a program of instructions executable by the processing device 51 to perform program steps as described herein according to the present principles.

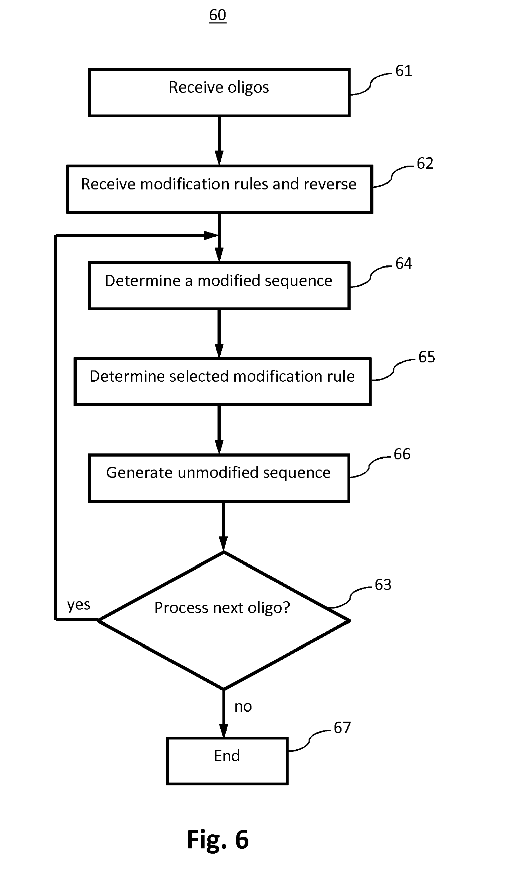

Referring to Fig. 6, one embodiment of a method 60 for determining unmodified sequences of quaternary code symbols from a plurality of oligos generated from modified code symbol sequences with code symbol values corresponding to nucleotide types is schematically shown. The method may, for example, be computer implemented, wherein the sequencing is performed using, e.g., a nucleic acid sequencer.

The presented method allows converting the oligos created using the method of creating a plurality of oligos with a targeted distribution of nucleotide types, represented by a plurality of sequences of quaternary code symbols, described above back to the unmodified sequences of quaternary code symbols. The method allows retrieving or extracting the unmodified sequences from the oligos corresponding to the modified sequences.

In the shown embodiment, in a first step 61 the plurality of oligos is received, for example through an interface to a nucleic acid storage. The oligos carry encoded in their succession of nucleotides the corresponding sequences of quaternary code symbols which had been modified depending on a targeted distribution of nucleotide types.

Further, in a second step 62 a set of modification rules and of reverse, i.e. inverse, modification rules is received. The modification rules had been used during modification before creation of the oligos. Each modification rule is adapted to unambiguously assign unmodified code symbol values, i.e. values of unmodified code symbols, to corresponding modified code symbol values, i.e. corresponding values of modified code symbols. The reverse modification rules are adapted to revert the modifications applied to code symbol sequences by the corresponding modification rules. In one embodiment, the set of reverse modification rules is not received but directly calculated from the received set of modification rules.

The following steps are repeated for each of the plurality of oligos. This is achieved, for example, by checking 63 after processing steps 64, 65 and 66 whether or not any next oligo remains to be processed and repeating the steps if more oligos remain to be processed.

In step 64 a modified sequence of quaternary code symbols is determined by acquiring and sequencing a corresponding oligo of the plurality of oligos.

In a next step 65 a selected modification rule of the set of modification rules that has been applied to generate the modified sequence is determined.

In a next step 66 an unmodified sequence of quaternary code symbols is generated from the modified sequence, i.e. a transformation is performed, wherein the modified code symbol values of the modified sequence are replaced with their assigned corresponding unmodified code symbol values according to a reverse modification rule adapted to revert changes that have been applied by the selected modification rule.

Depending on the result of the checking 63, steps 64, 65 and 66 are either repeated with a next oligo or the method proceeds to an end step 67. The end step 67 may, for example, refer to further processing, storing or decoding the quaternary code symbol sequences after removal of the applied modification, wherein decoding refers to retrieval of the user data initially encoded in the unmodified code symbol sequences.

For step 65 it is necessary to find out, which one of the four rules has been applied to the modified code symbol sequence determined from the processed oligo. This can be determined by investigating the code words contained in the modified code symbol sequence.

Irrespective whether the random, pseudo random or deterministic approach has been chosen for selecting the modulation rule for modulating the code symbol sequence, in one embodiment said modulation rule can be determined, for example, by checking the reconstructed code symbol sequences for correctness for all possible corresponding reverse (or inverse) modulation rules.

Referring to Fig. 7, one example embodiment of steps 65 and 66, i.e. determining of the selected modification rule and generating an unmodified sequence, is schematically illustrated. In a first sub step 71 a set of potential unmodified sequences based on reverse modification rules corresponding to all modification rules of the set of modification rules is generated.

In a second sub step 72 a compliance of the generated potential unmodified sequences with one or more constraints a correct unmodified sequence complies with before modification is evaluated. Examples for such constraints are the maximum length of a self-reverse complementary part of an oligo corresponding to the unmodified code symbol sequence, a maximum run length of identical code symbols within the unmodified code symbol sequence and a combination thereof.

In a third sub step 73 the selected modification rule is then selected as the modification rule that allows generating the correct unmodified sequence in compliance with the one or more constraints.

In other words, for determining the correct rule that has been applied to modify the original modified sequence, reverse or inverse modification rules, each corresponding to one of the modification rules, are provided, and all inverse rules are applied to a modified sequence of quaternary code symbols determined by sequencing an oligo to generate a corresponding set of potential unmodified sequences. Then it is checked, which of the resulting potential code symbol sequences contains only valid code words, as most probably only the correct rule will allow generation of a valid code symbol sequence.

In one embodiment, if the received oligos are not error free, the inverse rule allowing creation of a potential unmodified sequence with the maximum amount of valid code words is chosen as the rule that has been used as the selected modification rule.

In another embodiment where the selecting has been carried out pseudo randomly where the different modulation rules have been selected periodically according to a modulo 4 division of the number of the unmodified code symbol sequence, said selected modulation rule can be determined afterwards, if a consecutive numbering of the created oligo is encoded within the oligo. Here, the applied modulation rule can be determined from a modulo 4 division of said encoded oligo number.

In another embodiment where the selecting has been carried out according to a deterministic selection scheme, based on the content of the modified code symbol sequences, the unchanged attributes of the unmodified code symbol sequence are determined to find out which rule has been applied. As those attributes remained unchanged, it is possible to determine, which reverse modification rule shall be applied to do the reverse modification. For example, in one embodiment the frequency distribution of the number of pairs of neighboring code symbols with identical values corresponding to the frequency distribution shown in Fig. 2 is determined and divided into four intervals of integral areas of same size as shown in Fig. 3 to correctly assign the four rules. After that, the modification is removed from each modified code symbol sequence by determining the contained number of pairs and assigning the correct reverse modification rule.

In one embodiment, if a "number of pairs" value is found near to a limit of two rules and if errors introduced, e.g., by channel distortions lead to a wrong decision, the wrong decoding will be detected and then the most presumable rule is chosen. In these cases two rules have to be tried.

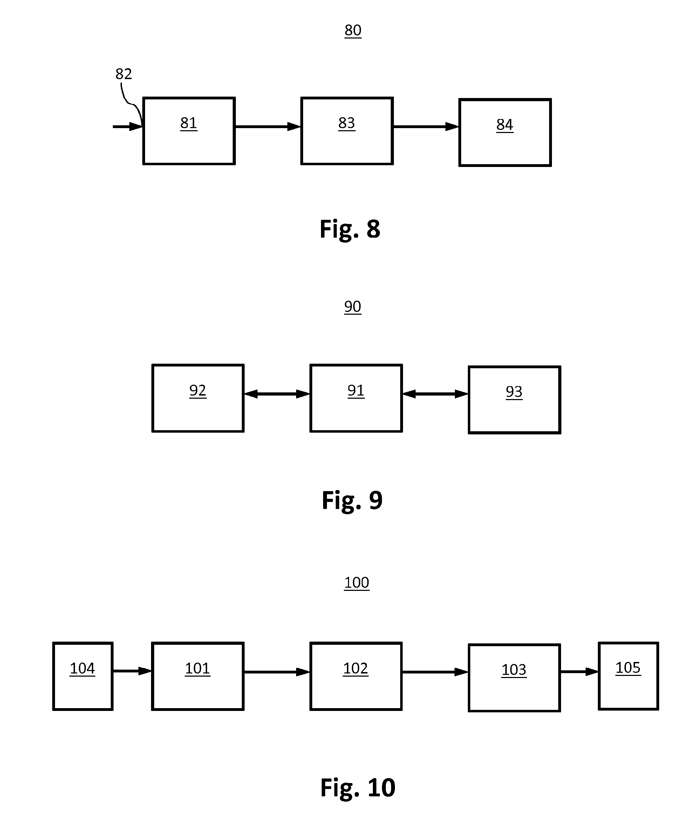

Accordingly, one embodiment of an apparatus 80 for determining unmodified sequences of quaternary code symbols from a plurality of oligos generated from modified code symbol sequences with code symbol values representing nucleotide types is schematically shown in Fig. 8. The apparatus 80 allows implementing the advantages and characteristics of the described method as part of an apparatus for determining unmodified sequences of quaternary code symbols from a plurality of oligos generated from modified code symbol sequences.

The apparatus 80 comprises a sequencer unit 81 configured to determine a modified code symbol sequence by sequencing a corresponding oligo of the plurality of oligos. The sequencer unit 81 comprises an input 82 configured to receive the plurality of oligos.

The apparatus 80 further comprises a modification rule determination unit 83 configured to determine a selected modification rule of a set of modification rules that has been applied to generate the modified code symbol sequence. The modification rules had been used during modification before creation of the oligos. Each modification rule is adapted to unambiguously assign unmodified code symbol values, i.e. values of unmodified code symbols, to corresponding modified code symbol values, i.e. corresponding values of modified code symbols. The set of modification rules are either received through an interface or stored in a memory connected to or comprised in the modification rule determination unit 83. Reverse modification rules adapted to revert the modifications applied to code symbol sequences by the corresponding modification rules are either received through an interface, too, or directly calculated from the received set of modification rules.

Further, the apparatus 80 comprises a modification removal unit 84 connected to receive the modified quaternary code symbol sequence and the reverse modification rule of the selected modification rule that is determined by the modification rule determination unit 83. The modification removal unit 84 is configured to generate, for each of at least some of the plurality of oligos, an unmodified sequence from the modified sequence, wherein the modified code symbol values of the modified sequence are replaced with their assigned corresponding unmodified code symbol values according to a reverse modification rule adapted to revert changes that have been applied by the selected modification rule.

In the embodiment shown in Fig. 8 the sequencer unit 81, the modification rule determination unit 83 and the modification removal unit 84 directly communicate with each other. In another embodiment the apparatus comprises a controller unit connected to one or more of the units and controls their communication.

Depending on the embodiment, the sequencer unit 81, the modification rule determination unit 83 and the modification removal unit 84 are provided as separate devices or jointly as one device. In one embodiment, the modification rule determination unit 83 and the modification removal unit 84 are provided as functionality carried out or implemented by a processor, microprocessor, microcontroller or other processing device, computer or other programmable apparatus connected to or comprising a memory and connected with the sequencer unit 81.

Another embodiment of an apparatus 90 for determining unmodified sequences of quaternary code symbols from a plurality of oligos generated from modified code symbol sequences with code symbol values corresponding to nucleotide types according to the present principles is schematically illustrated in Fig. 5. The apparatus 90 comprises a processing device 91, a nucleic acid sequencer device 92 and a memory device 93 storing instructions that, when executed, cause the apparatus to perform steps according to one of the described methods.

For example, the processing device 91 can be a processor adapted to perform the steps according to one of the described methods. In one embodiment according to the present principles, said adaptation comprises that the processor is configured, e.g. programmed, to perform steps according to one of the described methods for determining unmodified sequences of quaternary code symbols from a plurality of oligos generated from modified code symbol sequences with code symbol values corresponding to nucleotide types.

A part of the memory device 93 is a non-transitory program storage device readable by the processing device 91, tangibly embodying a program of instructions executable by the processing device 91 perform program steps as described herein according to the present principles.

Referring to Fig. 10, one embodiment of a nucleic acid storage system 100 is schematically illustrated. In the shown embodiment the synthesizer unit of an apparatus 101 for creating a plurality of oligos with a targeted distribution of nucleotide types, represented by a plurality of sequences of quaternary code symbols, each code symbol value representing a nucleotide type, is connected to a nucleic acid storage 102 configured to store the plurality of oligos synthesized by the synthesizer unit. In another embodiment the nucleic acid storage 102 is comprised in the apparatus 101, for example as part of the synthesizer unit.

In the embodiment shown in Fig. 10 an apparatus 103 for determining unmodified sequences of quaternary code symbols from a plurality of oligos generated from modified code symbol sequences with code symbol values representing nucleotide types is connected to receive these oligos from the nucleic acid storage 102. In another embodiment the nucleic acid storage 82 is part of the apparatus 103.

The nucleic acid storage 102, as well as the synthesizing process performed by the synthesizer unit of apparatus 101 and the sequencing process performed by the sequencer unit of apparatus 103 together can be considered a channel which may introduce errors, such as insertion or deletion errors or swaps, to the stored oligos or reconstructed code symbol sequences.

The embodiment of a nucleic acid storage system 100 shown in Fig. 10 further comprises a user data encoding unit 104 configured to receive input user data, e.g. as sequences of binary code symbols, and convert them into the plurality of sequences of quaternary code symbol values, i.e. the unmodified sequences, using a specific coding scheme which translates the input user data into sequences of code words, i.e. sequences of sequences of quaternary code symbols, wherein the values of the quaternary code symbols correspond to nucleotide types. The user data encoding unit 104 is configured to provide the unmodified sequences to the apparatus 101.

The embodiment of a nucleic acid storage system 100 shown in Fig. 10 further comprises a user data decoding unit 105 configured to receive the quaternary code symbol sequences after removal of the modulation by the apparatus 103 for determining unmodified sequences of quaternary code symbols from a plurality of oligos generated from modified code symbol sequences with code symbol values corresponding to nucleotide types. The data decoding unit 105 then performs the inverse of the operation performed by the user data encoding unit 104. The now restored unmodified sequences of quaternary code symbols are split into the code words again and converted back to the related user data.

Aspects of the present principles can be embodied as a method, an apparatus, a system, a computer program product or a computer readable medium, i.e. the present principles may be implemented in various forms of hardware, software, firmware, special purpose processors, or a combination thereof. Accordingly, aspects of the present principles can take the form of a hardware embodiment, a software embodiment or an embodiment combining software and hardware aspects. Preferably, the present principles are implemented as a combination of hardware and software. Aspects of the present principles may, for example, at least partly be implemented in a computer program comprising code portions for performing steps of the method according to an embodiment of the present principles when run on a programmable apparatus or enabling a programmable apparatus to perform functions of an apparatus or system according to an embodiment of the present principles. Moreover, the software is preferably implemented as an application program tangibly embodied on a program storage device. The application program may be uploaded to, and executed by, a machine comprising any suitable architecture. Preferably, the machine is implemented on a computer platform having hardware such as one or more processors/central processing units (CPU), a random access memory (RAM), and input/output (I/O) interface(s). The computer platform also includes an operating system and microinstruction code. The various processes and functions described herein may either be part of the microinstruction code or part of the application program (or a combination thereof), which is executed via the operating system. In addition, various other peripheral devices may be connected to the computer platform such as an additional data storage device and a printing device, as well as a synthesizer and/or a sequencer device.

Unless stated otherwise, terms such as "first" and "second" are used to arbitrarily distinguish between the elements the terms describe and are not necessarily intended to indicate temporal or other prioritization of the elements.

Any connection shown may be a direct connection or an indirect connection. Further, those skilled in the art will recognize that the boundaries between logic blocks are merely illustrative and that alternative embodiments may merge logic blocks or impose an alternate decomposition of functionality upon various logic blocks.

### CITATION LIST

[I] George M. Church, Yuan Gao, Sriram Kosuri, "Next-Generation Digital Information Storage in DNA", Science Vol. 337, 28 September 2012.
[II] Nick Goldman et al., "Towards practical, high-capacity, low-maintenance information storage in synthesized DNA", Nature Vol. 494, January 2013.
[III] "Low-Diversity Sequencing on the Illumnia HiSeq Platform", Illumina, Technical Note: DNA Sequencing, 20 August 2014.
[IV] "Using a PhiX Control for HiSeq@ Sequencing Runs", Illumina, Technical Note: Sequencing, 21 March 2013.

## Claims

1. Method (10) of creating a plurality of oligos with a targeted distribution of nucleotide types, represented by a plurality of sequences of quaternary code symbols, each code symbol value representing a nucleotide type, comprising for the plurality of sequences of quaternary code symbols:
- acquiring (14) an unmodified sequence from the plurality of sequences;
- selecting (15) a modification rule from a set of different modification rules, each adapted to unambiguously assign unmodified code symbol values to corresponding modified code symbol values;
- generating (16) a modified sequence from the unmodified sequence, wherein the unmodified code symbol values of the unmodified sequence are replaced with their assigned corresponding modified code symbol values according to the selected modification rule, if said unmodified values differ from said assigned corresponding modified values; and
- creating (17) an oligo of the plurality of oligos, comprising a nucleotide sequence corresponding to the modified sequence of quaternary code symbols.

2. Method according to claim 1, wherein said set of modification rules comprises 4 modification rules that differ from each other in that each unmodified code symbol value is assigned to a different corresponding modified code symbol value.

3. Method according to claim 1 or claim 2, wherein said selecting (15) comprises randomly selecting the modification rule from the set of different modification rules.

4. Method according to claim 1 or claim 2, wherein said selecting (15) comprises pseudo randomly selecting the modification rule from the set of different modification rules.

5. Method according to claim 1 or claim 2, wherein said selecting (15) comprises selecting the modification rule from the set of different modification rules based on an evaluation of a parameter of the unmodified code symbol sequence invariant to modification by any of the modification rules.

6. Method according to claim 5, wherein said parameter is a number of pairs of neighboring code symbols having identical code symbol values.

7. Method according to claim 5 or claim 6, wherein each of said modification rules is assigned to a different interval of values of said parameter, said different interval depending on a frequency distribution of said values of said parameter.

8. Method (60) of determining unmodified sequences of quaternary code symbols from a plurality of oligos generated from modified code symbol sequences with code symbol values representing nucleotide types, comprising for at least some of the plurality of oligos:
- determining (64) a modified sequence of quaternary code symbols by sequencing a corresponding oligo of the plurality of oligos;
- determining (65) a selected modification rule of a set of modification rules that has been applied to generate the modified sequence, each modification rule adapted to unambiguously assign unmodified code symbol values to corresponding modified code symbol values; and
- generating (66) an unmodified sequence of quaternary code symbols from the modified sequence, wherein the modified code symbol values of the modified sequence are replaced with their assigned corresponding unmodified code symbol values according to a reverse modification rule adapted to revert changes that have been applied by the selected modification rule.

9. Method according to claim 8, wherein the determining (65) of the selected modification rule and generating (66) an unmodified sequence comprises
- generating (71) a set of potential unmodified sequences based on reverse modification rules corresponding to all modification rules of the set of modification rules;
- evaluating (72) compliance of the potential unmodified sequences with one or more constraints a correct unmodified sequence complies with before modification; and
- determining (73) the selected modification rule as the modification rule that allows to generate the correct unmodified sequence in compliance with the one or more constraints.

10. Method according to claim 9, wherein the one or more constraints comprises at least one of a maximum length of a self-reverse complementary part of an oligo corresponding to the unmodified code symbol sequence, a maximum run length of identical code symbols within the unmodified code symbol sequence and a combination thereof.

11. Method according to claim 8, wherein said determining (65) of the selected modification rule comprises determining the selected modification rule from the set of different modification rules based on an evaluation of a parameter of the modified code symbol sequence invariant to modification by any of the modification rules.

12. Method according to claim 11, wherein said parameter is a number of pairs of neighboring code symbols having identical code symbol values.

13. Method according to claim 11 or claim 12, wherein each of said modification rules is assigned to a different interval of values of said parameter, said different interval depending on a frequency distribution of said values of said parameter, the method comprising
- evaluating at least some of the modified code symbol sequences to determine said frequency distribution of said values of said parameter;
- determining the different intervals assigned to the modification rules; and
- determining the selected modification rule by evaluating the modified code symbol sequence with respect to the different intervals.

14. Apparatus (40) for creating a plurality of oligos with a targeted distribution of nucleotide types, represented by a plurality of sequences of quaternary code symbols, each code symbol value representing a nucleotide type, comprising
- an acquisition unit (41) configured to acquire unmodified sequences from the plurality of sequences;
- a selection unit (43) configured to select, for each of the plurality of sequences of quaternary code symbols, a modification rule from a set of different modification rules, each adapted to unambiguously assign unmodified code symbol values to corresponding modified code symbol values;
- a modification unit (44) configured to generate a modified sequence from the unmodified sequence, wherein the unmodified code symbol values of the unmodified sequence are replaced with their assigned corresponding modified code symbol values according to the selected modification rule, if said unmodified values differ from said assigned corresponding modified values; and
- an oligo creation unit (45) configured to create an oligo of the plurality of oligos, comprising a nucleotide sequence corresponding to the modified sequence of quaternary code symbols.

15. Apparatus (80) for determining unmodified sequences of quaternary code symbols from a plurality of oligos generated from modified code symbol sequences with code symbol values representing nucleotide types, comprising
- a sequencer unit (81) configured to determine a modified code symbol sequence by sequencing a corresponding oligo of the plurality of oligos;
- a modification rule determination unit (83) configured to determine a selected modification rule of a set of modification rules that has been applied to generate the modified code symbol sequence, each modification rule adapted to unambiguously assign unmodified code symbol values to corresponding modified code symbol values; and
- a modification removal unit (84) configured to generate, for each of at least some of the plurality of oligos, an unmodified sequence from the modified sequence, wherein the modified code symbol values of the modified sequence are replaced with their assigned corresponding unmodified code symbol values according to a reverse modification rule adapted to revert changes that have been applied by the selected modification rule.


SEQUENCE LISTING

<110> Thomson Licensing

<120> Method and apparatus for creating a plurality of oligos with a
targeted distribution of nucleotide types

<130> PD150036-EPA

<140> EP15305791.4
<141> 2015-05-26

<160> 8

<170> BiSSAP 1.3

<210> 1
<211> 14
<212> DNA
<213> Artificial Sequence

<220>
<223> example 1 before application of rule #1

<400> 1
aaacaaagta aaac                                                    14


<210> 2
<211> 14
<212> DNA
<213> Artificial Sequence

<220>
<223> example 1 after application of rule #1

<400> 2
aaacaaagta aaac                                                    14


<210> 3
<211> 14
<212> DNA
<213> Artificial Sequence

<220>
<223> example 2 before application of rule #2

<400> 3
gataaataaa caat                                                    14


<210> 4
<211> 14
<212> DNA
<213> Artificial Sequence

<220>
<223> example 2 after application of rule #2

<400> 4
ctgtttgttt attg                                                    14

<210> 5
<211> 14
<212> DNA
<213> Artificial Sequence

<220>
<223> example 3 before application of rule #3

<400> 5
ataatgaaac aaat                                                         14


<210> 6
<211> 14
<212> DNA
<213> Artificial Sequence

<220>
<223> example 3 after application of rule #3

<400> 6
gcggcagggt gggc                                                         14


<210> 7
<211> 14
<212> DNA
<213> Artificial Sequence

<220>
<223> example 4 before application of rule #4

<400> 7
taagaaatat aaac                                                         14


<210> 8
<211> 14
<212> DNA
<213> Artificial Sequence

<220>
<223> example 4 after application of rule #4

<400> 8
acctcccaca cccg                                                         14